(19) Europäisches Patentamt
European Patent Office
Office européen des brevets

(11) Publication number: **0 208 456 B1**

(12) **EUROPEAN PATENT SPECIFICATION**

(45) Date of publication of patent specification: **06.06.90**

(51) Int. Cl.[5]: **C 07 C 209/30,** C 07 C 211/51

(21) Application number: **86304838.5**

(22) Date of filing: **24.06.86**

(54) **Process for making n-monosubstituted p-phenylenediamines.**

(30) Priority: **10.07.85 US 753742**

(43) Date of publication of application:
**14.01.87 Bulletin 87/03**

(45) Publication of the grant of the patent:
**06.06.90 Bulletin 90/23**

(84) Designated Contracting States:
**AT BE CH DE FR GB IT LI LU NL SE**

(56) References cited:
**US-A-4 313 002**

**CHEMICAL ABSTRACTS, vol. 66, no. 9, 27th February 1967, page 3559, column 2, abstract no. 37540q, Columbus, Ohio, US; N.S. KOZLOV et al.: "Catalytic reduction of nitroso-, azo-, azoxy-, and hydrazo-benzenes, and phenylhydroxylamine by alcohols" & UCH. ZAP. PERM. GOS. PEDAGOG INST. 1965, 32, 87-94**

**CHEMICAL ABSTRACTS, vol. 100, 18th-25th June 1984, page 547, column 2, abstract no. 209264s, Columbus, Ohio, US; G.A.**

(73) Proprietor: **UNIROYAL CHEMICAL COMPANY, Inc.**
**World Headquarters**
**Middlebury Connecticut 06749 (US)**

(72) Inventor: **Batorewicz, Wadim**
**99 Whalley Avenue**
**New Haven Connecticut 06515 (US)**

(74) Representative: **Harrison, Michael Robert et al**
**URQUHART-DYKES & LORD 5th Floor Tower House Merrion Way**
**Leeds LS2 8PA West Yorkshire (GB)**

(56) References cited:

**ZAPEVALOV et al.: "Products of the reaction of p-nitrosodiphenylamine with triisobutylaluminum" & DOKL. AKAD. NAUK SSSR 1984, 274(2), 335-338**

**JOURNAL OF THE CHEMICAL SOCIETY, vol. B, 1968, page 191-195, London, GB; J. HUTTON et al.: "The mechanism of reduction of nitrosobenzene by alcohols"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 208 456 B1**

Courier Press, Leamington Spa, England.

## Description

### Background of the Invention

It has long been desired to develop an efficient and economical process for the manufacture of N-monosubstituted p-phenylenediamines. These materials are particularly useful for the manufacture of antioxidants and antiozonants for use in rubber products and as intermediates for the preparation of dyes.

Conventionally, these materials have been prepared by the hydrogenation of nitrosoarylamines as a salt of an alkali metal in an aqueous system. Reduction is typically performed using gaseous hydrogen in the presence of a conventional hydrogenation catalyst. An example of this process is shown in U.S. Patent 2,974,169.

In later work, such as described in U.S. Patent 4,313,002, the alkaline metal salts are reduced in other solvent systems such as aromatic hydrocarbons and saturated aliphatic alcohols. Still the hydrogenation step is taught to be performed using hydrogen over a conventional hydrogenation catalyst.

These processes suffer from the disadvantage of utilizing hydrogenation catalysts which are typically very costly noble metal catalysts such as, for example, platinum and palladium.

U.S. Patent 4,448,994 teaches the use of a reducing sugar to reduce an alkalkine metal salt of a nitrosoamine in an aqueous solution. This process suffers from the typical problems associated with aqueous solutions of these salts in that they tend to hydrolyze and, therefore, are unstable upon storage.

In U.S. Patent 4,479,008, it is disclosed that a diphenylamine can be N-nitrosated; rearranged to form 4-nitrosodiphenylamine and subsequently hydrogenated over a conventional 5% palladium on carbon catalyst with a hydrogen pressure of 34 to 54 bar (500—800 psi) at 80°C by using an aliphatic alcohol as a medium for all of the foregoing steps. This disclosure teaches the necessity of utilizing the noble metal catalysts of the prior art for the reduction step which, as noted above, are extremely expensive.

### Summary of the Invention

In accordance with the invention, it has been discovered that N-mono-substituted p-phenylenediamines can be prepared by reacting 4-nitrosoanilines with alcohol under basic conditions in the absence of hydrogen and expensive noble metal catalyst. The reaction can be performed in an alcoholic solution of the same alcohol which is used as the reducing agent. In fact, all of the conventional steps usually associated with the production of 4-mono-substituted p-phenylenediamines from N-mono-substituted anilines, i.e., the N-nitrosation followed by the rearrangement to the 4-nitroso intermediate can be performed using the same alcohol which in this invention can function as the reducing agent. This process results in good yields of the desired end products with little azo and azoxy by-product formation. In addition, the N-mono-substituted p-phenylenediamine is readily separated at high levels of purity.

It is most surprising that the process of the invention would operate to produce the N-mono-substituted p-phenylenediamine. Work previously performed on nitrosobenzenes and alcohol taught that the reduction, in the presence of a base, resulted in almost quantitative yields of azoxybenzene, rather than the corresponding aminobenzene. For example, *Hutton et al in "The Mechanism of Reduction ot Nitrosobenzene by Alcohol"*, J.P. Chem. Soc. (B) 1968, pages 191—195 report this reaction. The mechanism is described on page 191 and exemplified on page 195. Interestingly, even in the heretofore mentioned patent, where the alkali metal salts of nitrosodiphenylamine were in the presence of alcohol (U.S. Patent No. 4,313,002) it was deemed essential to effect the reduction by means of hydrogen and the conventional noble metal catalysts.

### Detailed Description of the Invention

The process of the invention may be described schematically as follows:

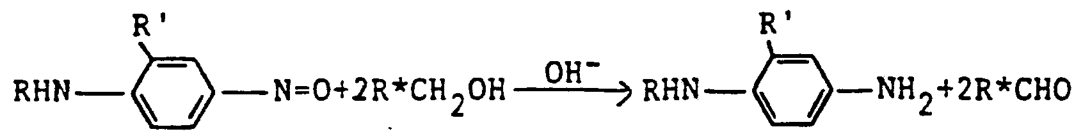


wherein R is a $C_1$—$C_{12}$ alkyl, phenyl, naphthyl, or phenyl substituted in the 2-, 4- or 2,4- positions by $C_1$—$C_8$ alkyl; R′ is hydrogen or a $C_1$—$C_6$ alkyl; and R* is hydrogen, $C_1$—$C_{12}$ alkyl or phenyl.

Preferably, in the process of this invention, the alcohol has the formula R″OH wherein R″ is $C_1$—$C_{12}$ alkyl or benzyl.

More preferably, the R in the above-described process is $C_3$—$C_8$ alkyl or phenyl, and R′ is hydrogen or $C_1$—$C_4$ alkyl.

Most preferably, R″ is $C_4$—$C_6$ alkyl.

Of the aforesaid N-mono-substituted p-phenylenediamine, the most important product is p-aminodiphenylamine. This material is useful for the preparation of well-known antioxidants; the N-alkyl-N-phenyl p-phenylenediamines. However, the process may also be readily applied to the preparation of N-isopropyl p-phenylenediamine; N-1-methylethyl p-phenylenediamine; N-cyclohexyl p-phenylenediamine; N-1,3-dimethylbutyl p-phenylenediamine; and N-1,4-dimethylpentyl p-phenylenediamine.

The alcohols which may act as a reducing agent in accordance with the teaching of the invention include the primary and secondary saturated alcohols having up to twelve carbon atoms. Examples include butanols, pentanols, hexanols, heptanols, octanols, nonanols, decanols and benzyl alcohol. Tertiary alcohols cannot be used since the presence of the alpha-hydrogen is necessary for the alcohol to act as a reducing agent.

Those skilled in the art will understand that the corresponding p-nitrosodiphenylamine may be readily selected to form the desired end product. While 4-nitrosodiphenylamine is the preferred starting material, depending on the desired end product, the following amines may also be used: 2,2′-diisopropyl, 2,2′-diisobutyl, 2,2′-di-n-butyl, 2,4′-diisopropyl; 2-isopropyl-4′-isobutyl, 4′-isopropyl, 4′-n-propyl, 4′-isobutyl, 4′-n-butyl, 4′-n-pentyl, 4′-isopentyl, 4′-n-hexyl, 4′-(2-ethylhexyl), 2,2′-diisopropyl-4′-n-butyl and 2,2′-diisobutyl-4′-n-hexyl.

The 4-nitroso-M-mono-substituted aniline can be obtained by the rearrangement of the N-nitroso derivative by contact with a mineral acid such as a hydrogen halide. This rearrangement is well known to those skilled in the art and clearly described in U.S. Patent 4,313,002 and U.S. Patent 4,479,008. It will be understood that, after the rearrangement it is not necessary to isolate the intermediate products.

Although other basic materials may be employed, generally, the reaction is performed in the presence of an alkali metal hydroxide.

Most preferably, the alkali metal is sodium or potassium. Sufficient base should be added to cause the reaction to go to completion and to suppress formation of azo and azoxy by-products. The base (OH)/nitroso compound (NO) molar ratio which is at least 0.75:1, is preferably from 1:1 to 2:1, and most desirably from 1.25:1 to 1.5:1.

The molar ratio of the alcohol to the nitroso compound (ROH/NO) which may be from 2:1 to 100:1, is preferably 5:1 to 50:1 and most desirably 7.5:1 to 20:1. Enough alcohol should be present not only to reduce the nitroso compound, but also to serve as the solvent for the reaction. Generally speaking, from 20 to 30 weight percent of the nitroso compound in the alcohol is a practical concentration.

Alcohols having three or more carbon atoms are preferable to methanol and ethanol. In addition, it is desirable to minimize the water present in the process to avoid hydrolysis of the alkali metal salt. This can be done by the addition of the base in the form of a solid, rather than in the form of an aqueous solution. However, for convenience, the base may be added to the alcoholic medium in a water solution, preferably at a 50% by weight concentration.

The reaction temperature may range broadly from 50 to 200°C, but is preferably maintained between 100 and 150°C. Generally speaking, it is most convenient to carry out the reaction at atmospheric pressure, however, for lower boiling alcohols, it may be desirable to operate under higher pressures.

It is not fully understood exactly why the nitroso compounds of the instant invention behave differently than the nitrosobenzenes discussed in the Hutton et al article. However, as will be observed from the following experiments, the results are unexpectedly different. It is believed that during the reaction the alcohol is oxidized to be corresponding aldehyde or ketone, but such reaction mechanisms are of little importance in realizing the advantages of the instant invention.

To illustrate further, the invention, the following examples are set forth. In all instances, the reaction mixtures were evaluated by infrared analysis and high pressure liquid chromatography (HPLC) for the presence of the nitroso starting material and azo or azoxy by-products, such as 4,4′-bis (N-monosubstituted amino) azoxy- and azo-benzenes. In all cases, only about 1 to 2% of each, i.e., azo and azoxy by-products, were detected.

## Example 1

Preparation of N-phenyl-p-phenylenediamine

To a 1 liter three-necked round bottom flask fitted with a stirrer, a condenser and a thermometer were added 99.0 (0.5 mole) N-phenyl-4-nitrosoaniline, 254 g n-hexanol and 40 g (0.5 mole) aqueous NaOH (50% by weight). The dark reddish brown solution was stirred, heated to 100°C, at which point an exotherm developed, and heating was discontinued. A temperature rise to 120°C was observed within 15 minutes. After another 15 minutes, the temperature returned to 100°C. Stirring was continued for one hour while maintaining the reaction mixture at 100—105°C. Thereafter, a sample of the reaction mixture was analyzed by HPLC; no nitroso starting material was detected.

After cooling to room temperature, the reaction mixture was washed thoroughly with water, the excess alcohol distilled off at a temperature of 165—170°C and at a pressure of 13.3—66.7 Pa, and the product was isolated by vacuum distillation. 77.1 g of light tan-colored oily product was obtained (83.8% yield), which crystallized upon cooling. Gas chromatography indicated a product purity of 98%.

Additional experiments, reported in Table 1 below, were performed following essentially the above procedure, except for the variations noted:

TABLE I

| RUN NO. | 1 | 2 | 3 | 4 | 5 | 6 | 7 | 8 | 9 | 10 |
|---|---|---|---|---|---|---|---|---|---|---|
| Alcohol | MEOH | MEOH | IPOH | IPOH | IPOH | MBOH | TPOH | MPOH | CHOH | ETGL |
| Reaction Temperature °C | ca. 65* | 115—125 | ca. 82* | 115—125 | ca. 107* | 115* | ca. 102* | 100—105 | 100—105 | 100—105 |
| Reaction Period, hrs. | 1.5 | 5.7 | 2.0 | 2.0 | 3.0 | 1.5 | 5.0 | 1.0 | 1.5 | 3.0 |
| RESULTS | | | | | | | | | | |
| NPA (1) Remaining % | 100 | 50 | 100 | 0 | 0 | 0 | 100 | 0 | 0 | 16 |
| NPPD (2) Product % | — | 50 | — | 76 | 89 | 88 | — | 66 | 80 | 79 |

* Conducted at reflux temperature.

MEOH = methanol
IPOH = 2-propanol
MBOH = 3-methyl-1-butanol
MPOH = 4-methylpentan-2-ol

TPOH = tertiary pentanol
IBOH = isobutyl alcohol
CHOH = cyclohexyl alcohol
ETGL = ethylene glycol

(1) N-phenyl-4-nitrosoaniline
(2) N-phenyl-p-phenylenediamine

Example 2

Preparation of N-isopropyl-p-phenylenediamine

Following essentially the procedure of Example 1, 66.0 g (0.25 mole) N-isopropyl-4-nitrosoaniline, 20 g (50%) sodium hydroxide and 264 g n-hexanol were kept at 100°C for 1.5 hours. HPLC analysis of the reaction mixture indicated absence of the nitroso starting compound. By vacuum distillation (95—101°C at 13.3—26.6 Pa), 31.3 g of product were recovered (48.6 wt.% yield).

Example 3

Preparation of N-cyclohexyl-p-phenylenediamine

51.0 g (0.25 mole) N-cyclohexyl-4-nitrosoaniline, 20.0 g (50%) NaOH and 135 g n-hexanol were charged to a 0.5 liter three-necked round bottom flask equipped with stirrer, condenser and thermometer, and heated while stirring to 95°C until the development of an exotherm was observed. Heating was discontinued, the reaction temperature continued to rise to 115°C and then dropped, within 10 minutes, to 100°C. With the application of heat, a temperature of 90—95°C was maintained for 30 minutes. The reaction mixture contained no unreacted nitroso compound. The yield of the product was 64 wt.%; m.p. 59—61°C.

Example 4

Preparation of 2,2'-Diisopropyl-N-phenyl-p-phenylenediamine

2,2'-diisopropyl-N-phenyl-4-nitrosoaniline (40.0 g, 0.167 mole), sodium hydroxide (50%; 14.0 g, 0.17 mole) and n-hexanol (120 g) were added to a reactor as described in Example 3 and, with agitation, heated to 100—105°C for one hour. Infrared and HPCL analyses of the reaction mixture indicated incomplete conversion. The reaction mixture was heated at 125°C for 3.5 hours, cooled and washed several times with water. Quantitative gas chromatographic analysis of the hexanol solution indicated 73 wt.% yield.

The above embodiments and examples illustrate the scope and spirit of the instant invention. These embodiments and examples will make apparent, to those skilled in the art, other embodiments and examples. These other embodiments and examples are within the contemplation of this invention. Therefore, the instant invention should be limited only by the appended claims.

**Claims**

1. A process for preparing an N-mono-substituted p=phenylenediamine characterised in that the process comprises reacting a compound having the formula:

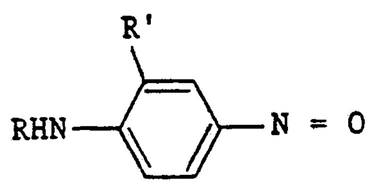

wherein R is a $C_1$—$C_{12}$ alkyl, phenyl, naphthyl, or phenyl substituted in the 2-, 4- or 2,4- positions by $C_1$—$C_8$ alkyl; and R' is hydrogen or $C_1$—$C_6$ alkyl with a primary or secondary alcohol at a temperature between 50° and 200°C wherein the molar ratio of alcohol to nitroso compound is from 2:1 to 100:1, in the presence of a base, wherein the molar ratio of OH:nitroso compound is at least 0.75:1, in the absence of hydrogen and noble metal catalyst.

2. A process according to claim 1 characterised in that said alcohol has the formula R''OH wherein R'' is $C_1$—$C_{12}$ alkyl or benzyl, and said base is an alkali metal hydroxide.

3. A process according to any of the preceding claims characterised in that R is $C_3$—$C_8$ alkyl or phenyl, and R' is hydrogen or $C_1$—$C_4$ alkyl.

4. A process according to any of the preceding claims characterised in that R'' is $C_4$—$C_6$ alkyl, and said base is NaOH or KOH.

5. A process according to any of the preceding claims characterised in that the reaction temperature is from 100 to 150°C.

**Patentansprüche**

1. Verfahren zur Herstellung eines N-monosubstituierten p-Phenylendiamins, dadurch gekennzeichnet, daß bei dem Verfahren eine Verbindung mit der Formel

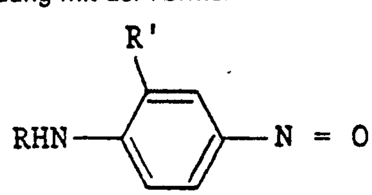

worin R eine $C_1$- bis $C_{12}$-Alkylgruppe, eine Phenylgruppe, eine Naphthylgruppe oder eine Phenylgruppe ist, die in der 2-, der 4- oder der 2- und 4-Stellung durch eine $C_1$- bis $C_8$-Alkylgruppe substituiert ist, und R' ein Wasserstoffatom oder eine $C_1$- bis $C_6$-Alkylgruppe ist, bei einer Temperatur zwischen 50 und 200°C mit einem primären oder sekundären Alkohol, wobei das Molverhältnis von Alkohol zu Nitrosoverbindung 2:1 bis 100:1 beträgt, in Gegenwart einer Base, wobei das Molverhältnis von OH zu Nitrosoverbindung wenigstens 0,75:1 beträgt, in Abwesenheit von Wasserstoff und Edelmetallkatalysator umgesetzt wird.

2. Verfahren nach Anspruch 1, dadurch gekennzeichnet, daß der Alkohol die Formel R''OH hat, worin R'' eine $C_1$- bis $C_{12}$-Alkylgruppe oder eine Benzylgruppe ist, und daß die Base ein Alkalimetallhydroxid ist.

3. Verfahren nach einem der vorhergehenden Ansprüche, dadurch gekennzeichnet, daß R eine $C_3$- bis $C_8$-Alkylgruppe oder eine Phenylgruppe ist und R' ein Wasserstoffatom oder eine $C_1$- bis $C_4$-Alkylgruppe ist.

4. Verfahren nach einem der vorhergehenden Ansprüche, dadurch gekennzeichnet, daß R'' eine $C_4$- bis $C_6$-Alkylgruppe ist und die Base NaOH oder KOH ist.

5. Verfahren nach einem der vorhergehenden Ansprüche, dadurch gekennzeichnet, daß die Reaktionstemperatur 100 bis 150°C beträgt.

**Revendications**

1. Procédé pour la préparation d'une p-phénylenediamine N-monosubstituée caracterisé en ce que le procédé comprend la réaction d'un composé ayant pour formule:

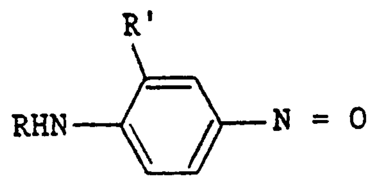

où R est alkyle $C_1$—$C_{12}$, phényle, naphtyle ou phényle substitué aux positions 2-, 4- ou 2,4- par alkyle $C_1$—$C_8$; R' est hydrogène ou alkyle $C_1$—$C_6$, avec un alcool primaire ou secondaire à une température comprise entre 50° et 200°C où le rapport molaire de l'alcool au composé nitroso est compris entre 2:1 et 100:1, en présence d'une base, où le rapport molaire de OH: composé nitroso est d'au moins 0,75:1, en l'absence d'hydrogène et d'un catalyseur de métaux nobles.

2. Procédé selon la revendication 1, caractérisé en ce que ledit alcool a pour formule R''OH où R'' est alkyle $C_1$—$C_{12}$ ou benzyle, et ladite base est un hydroxyde d'un métal alcalin.

3. Procédé selon l'une quelconque des revendications précédentes, caractérisé en ce que R est alkyle $C_3$—$C_8$ ou phényle et R' est hydrogène ou alkyle $C_1$—$C_4$.

4. Procédé selon l'une quelconque des revendications précédentes, caractérisé en ce que R'' est alkyle $C_4$—$C_6$ et ladite base est NaOH ou KOH.

5. Procédé selon l'une quelconque des revendications précédentes, caractérisé en ce que la température de la réaction est comprise entre 100 et 150°C.